# EUROPÄISCHE PATENTSCHRIFT

(11) **EP 2 182 488 B1**
(45) Veröffentlichungstag und Bekanntmachung des Hinweises auf die Patenterteilung: **08.08.2012**
(21) Anmeldenummer: 09013578.1
(22) Anmeldetag: 28.10.2009
(51) Int. Cl.: G06T 7/00, A61C 13/00

(54) **Gebissbild-Simulationsvorrichtung**
Dentition image simulator
Dispositif de simulation d'une image de dentition

(30) Priorität: 28.10.2008 DE 202008014344 U
(43) Veröffentlichungstag der Anmeldung: 05.05.2010
(60) Teilanmeldung: 11008543.8
(73) Patentinhaber: Edinger-Strobl, Verena, 6020 Innsbruck (AT)
(72) Erfinder: Edinger-Strobl, Verena, 6020 Innsbruck (AT)
(74) Vertreter: Thoma, Michael

(56) Entgegenhaltungen:
- EP-A1- 1 304 088
- WO-A1-2008/031614
- DE-A1-102005 023 875
- US-A1- 2003 198 384
- US-A1- 2004 197 727
- US-A1- 2005 123 180
- US-A1- 2007 129 991
- LUCCHESE L ET AL: "Colour image segmentation: a state-of-the-art survey", PROCEEDINGS OF THE INDIAN NATIONAL SCIENCE ACADEMY. PART A.PHYSICAL SCIENCES, INDIAN NATIONAL SCIENCE ACADEMY, NEW DEHLI, IN, Bd. 67, Nr. 2, 1. März 2001 (2001-03-01), Seiten 207-221, XP002244932, ISSN: 0370-0046
- HARALICK R M ET AL: "IMAGE SEGMENTATION TECHNIQUES", COMPUTER VISION GRAPHICS AND IMAGE PROCESSING, ACADEMIC PRESS, DULUTH, MA, US, Bd. 29, Nr. 1, 1. Januar 1985 (1985-01-01) , Seiten 100-132, XP000974960,
- MCINERNEY T ET AL: "Deformable Models in Medical Analysis: A Survey", MEDICAL IMAGE ANALYSIS, OXFORDUNIVERSITY PRESS, OXFORD, GB, Bd. 1, Nr. 2, 1. Juni 1996 (1996-06-01), Seiten 91-108, XP002230283, ISSN: 1361-8423, DOI: DOI:10.1016/S1361-8415(96)80007-7

## Beschreibung

Die vorliegende Erfindung betrifft eine Gebissbild-Simulationsvorrichtung zur Simulation eines Gebissbilds zur Behandlungsergebnis-Visualisierung und/oder Generierung von Daten für die Herstellung von Zahnersatzteilen, mit einer Ist-Bilderzeugungsvorrichtung zur Erzeugung einer Ist-Darstellung des Gebisses in seinem Ist-Zustand, einer Bild-Manipulationsvorrichtung zur Manipulation von Bilddaten der Ist-Darstellung anhand einer vorgebbaren Gebiss- und/oder Zahnbehandlungsfunktion, und einer Soll-Bilderzeugungsvorrichtung zur Erzeugung einer Soll-Darstellung des Gebisses in seinem Soll-Zustand anhand der Ist-Darstellung und der manipulierten Bilddaten.

Bei Zahnbehandlungen und der Herstellung von Zahnersatz ist es bislang trotz aller bekannter zahnärztlicher Hilfsmittel schwierig, vorab den Zahnersatz bzw. die Zahnveränderung, beispielsweise durch Abschleifen, Bleichen, Überkronen und dergleichen, genau passend zu konfigurieren, so dass ein stimmiges Gebissbild entsteht, in dem ein beispielsweise einzusetzender Ersatzzahn sich passgenau in die vorhandene Zahnlücke einfügt und optisch mit den restlichen Zähnen harmoniert und nicht zuletzt auch dem Patienten gefällt. Hierzu ist es bekannt, Behandlungsergebnisse mittels Vorher-/Nachher-Bilder anderer Patienten, die eine ähnliche Behandlung hatten, zu visualisieren. Weiters gibt es Schaumodelle, die verschiedene Behandlungsalternativen vorzeigbar machen. Es gibt weiters Computerprogramme zur Demonstration verschiedener Behandlungen an virtuellen Modellen oder mit Bildsequenzen oder Videos von Fällen anderer Patienten.

Dies ist jedoch nur bedingt hilfreich, da es nicht individuell an die Gegebenheiten des jeweiligen Patienten angepasst ist. Durch Anfertigung von Provisorien lässt sich in manchen Fällen das Behandlungsziel vorab visualisieren, dies führt jedoch zu zusätzlichen Kosten und ist zeitraubend. In manchen Fällen ist das Ergebnis der Behandlung überhaupt nicht oder nur sehr schwer für den Patienten vorstellbar und vor der Behandlung am Patienten nicht visualisierbar, so z. B. bei Zahnaufhellungen, Füllungstherapien, Kieferorthopädischen Behandlungen, Kieferchirurgischen Behandlungen. Es wäre deshalb wünschenswert, das Ergebnis einer jeweiligen Zahnbehandlungsfunktion möglichst detailgetreu vor Behandlungsbeginn simulieren und visualisieren zu können, so dass der Zahnarzt gemeinsam mit dem Patienten die optimale Konfiguration des beispielsweise einzusetzenden Ersatzzahnes festlegen kann.

Hiervon abgesehen ist die Auswahl der passenden Zahnbehandlung wie z.B. des passenden Zahnersatzes für den Zahnarzt bzw. den Zahntechniker eine zunehmend mühsame Tätigkeit, da der zur Verfügung stehende Auswahlpool hierfür zunehmend größer wird. Es gibt z. B. Ersatzzahnrohlinge bzw. -materialien in unterschiedlichsten Formvarianten und Farbtonnuancen, die an sich alle durchgesehen werden müssten, um den optimal passenden Zahnersatz auszusuchen bzw. den nächstkommenden Zahnersatzrohling bzw. das nächstkommende Material zu wählen, der bzw. das den Umfang der notwendigen Nacharbeit minimiert und ein bestmögliches Ergebnis ermöglicht.

Weiterhin wurde bereits versucht, für die CAD-CAM Fertigung von Zahnersatz die Erfassung der Umrisskonturen des zu ersetzenden Zahnes bzw. die Bestimmung der Konturdaten des zu fertigenden Zahnes zu automatisieren. Hierzu werden in der Regel komplizierte dreidimensionale Erfassungsverfahren eingesetzt wie beispielsweise Laserscannen oder Lichtebenenschnittverfahren, die dreidimensionale Konturdaten generieren, anhand derer automatische Fertigungswerkzeuge wie CAM-Fräser gesteuert werden. Derartige dreidimensionale Zahnerfassungsvorrichtungen verarbeiten jedoch eine Unmenge an Daten und führen eine Vielzahl von Berechnungsschritten aus, was eine äußerst leistungsfähige Hardware erfordert, die in Arztpraxen regelmäßig nicht zur Verfügung steht.

Aus der Schrift US 2003/0198384 ist ein Verfahren zur Simulation und Visualisierung des Bleichens eines Gebisses auf Basis eines Gebissbildes bekannt, wobei Zähne des Gebisses im Ist-Bild automatisch segmentiert werden, wobei ihre Farbe dann entsprechend einer vorgesehenen Bleichbehandlung verändert und das veränderte Bild wieder als Soll-Bild angezeigt wird_{.}

Die Schrift US 2004/197727 offenbart die Simulation und Visualisierung einer kieferorthopädischen Behandlung auf Basis eines aufgenommenen, dreidimensionalen Modells des Gebisses. Die Zähne des Gebisses werden in diesem Ist-Bild segmentiert, ihre Lage wird entsprechend dem Behandlungsplan verändert und ein resultierendes Soll-Bild wird angezeigt. Ein ähnliches Verfahren zur Visualisierung und Veränderung eines Zahnbildes offenbart die Schrift US 2007/0129991.

Die Schrift Lucchese L. et al.: "Color Image Segmentation: A State-of-the-Art Survey" in Proceedings of the Indian National Science Academy, Band 67, Nr. 2 vom 01.03.2001, S. 207-221 beschreibt die Bearbeitung und Veränderung von Farbildern mittels Segmentierung und Bereichswachstum einzelner Segmente. In ähnlicher Weise beschreibt die Schrift Haralick R. M. et al. "Image Segmentation Techniques" in Computer Vision Graphics and Image Processing Band 29 Nr. 1 vom 01.01.1985, S. 100-132 verschiedene Bildsegmentationsverfahren im Überblick und nennt ebenfalls das Bereichswachstumsverfahren zur Bestimmung von Bildbereichsgrenzen. Die Schrift McInerney, T. et al "Deformable models in medical image analysis: a survey" in Medical Image Analysis Band 1 Nr. 2 vom 01.06.1996, S. 91-108 beschreibt die Anwendung von dynamisch verformbaren Bildmodellen zur Analyse von medizinischen Bildern wie Röntgenbildern des Knochengerüsts.

Die Schrift US 2005/123180 beschreibt ferner ein Segmentationsverfahren zur Lokalisierung eines Zahnes in einem digitalen Gebissbild. Die Segmentation wird hierbei durch Analyse und Vergleich einzelner Pixel ausgeführt.

Die Schrift WO 2008/031614 beschreibt ein Verfahren zur Auswahl von Zahnersatzteilen aus einer Datenbank anhand von zuvor bestimmten Daten der zu ersetzenden Zähne, wobei ausgewählte Zahnersatzteile mit Hilfe einer zuvor aufgenommenen Darstellung des Patientengesichts und -gebisses visualisiert werden.

Schließlich zeigt die Schrift EP 13 04 088 A1 ein Verfahren und eine Vorrichtung zum Herstellen von Passkörpern zur Restauration von Zähnen, wobei an einem Patienten bestimmte morphologische Parameter, die die Form und Größe des gewünschten Passkörpers festlegen, mit Konfektionsparametern einer Datenbank für Zahnteile verglichen werden, so dass eine notwendige Formkorrektur aus dem Vergleich bestimmt werden kann.

Hiervon ausgehend liegt der vorliegenden Erfindung die Aufgabe zugrunde, eine verbesserte Gebissbild-Simulationsvorrichtung zu schaffen, die Nachteile des Standes der Technik vermeidet und Letzteren in vorteilhafter Weise weiterbildet. Vorzugsweise soll mit einfachen Mitteln bei geringen zu verarbeitenden Datenmengen eine möglichst detailgetreue Gebissbild-Simulation und -Visualisierung erzielt werden, die das Ergebnis einer Zahnbehandlung eines bestimmten Patienten im voraus, d.h. schon vor Durchführung der Zahnbehandlung wiedergibt, um dem Patienten und Arzt eine Entscheidungshilfe zu geben.

Erfindungsgemäß wird diese Aufgabe durch eine Gebissbild-Simulationsvorrichtung gemäß Anspruch 1 gelöst. Bevorzugte Ausgestaltungen der Erfindung sind Gegenstand der abhängigen Ansprüche.

Es wird also vorgeschlagen, in der erzeugten Ist-Darstellung des Gebisses den zu behandelnden Zahn bzw. die zu behandelnden Zähne und/oder den zu behandelnden Gebissteil mittels einer intelligenten Segmentierungsvorrichtung automatisch in Abhängigkeit der vorgegebenen Zahnbehandlungsfunktion und ggf. weiterer individueller Patientenmerkmale wie bspw. Geschlecht, Alter, Kieferform, Lachlinie und dgl. zu segmentieren und den entsprechenden Bildabschnitt in Form von Pixeln zu identifizieren bzw. auszuschneiden, so dass sozusagen der die der Zahnbehandlung unterliegenden Zähne zeigende Bildausschnitt isoliert wird. Je nachdem, welche Zahnbehandlungsfunktion vorzunehmen ist, wird dann dieser isolierte Bildausschnitt manipuliert, indem die Pixeldarstellung in Abhängigkeit der vorzunehmenden Zahnbehandlung transformiert wird, während die restliche Ist-Darstellung des Gebisses unverändert bleibt, wodurch detailliert und realitätsnah die Auswirkung der vorzunehmenden Zahnbehandlung visualisiert werden kann. Als Ist- und Soll-Darstellungen werden dabei insbesondere zweidimensionale Bilder bzw. Darstellungen verwendet, die zur Visualisierung des Ergebnisses der geplanten Behandlung auf einem Bildschirm ausreichenden sind und die verarbeitete Datenmenge signifikant reduzieren. Erfindungsgemäß umfasst die Bildmanipulationsvorrichtung der Gebissbildsimulationsvorrichtung eine Segmentierungsvorrichtung zur automatischen Segmentierung eines Zahns und/oder eines Gebissteils in der Ist-Darstellung in Abhängigkeit der vorgegebenen Zahnbehandlungsfunktion sowie einen Segmentmanipulator zur automatischen Manipulation des segmentierten Bildausschnitts in Abhängigkeit der vorgegebenen Zahnbehandlungsfunktion. Die vorzunehmende Zahnbehandlungsfunktion kann dabei grundsätzlich unterschiedlicher Natur sein. Insbesondere kann die Zahnbehandlungsfunktion das Einsetzen eines Ersatzzahnes, mehrerer Ersatzzähne oder eines ganzen Gebissteiles sein, wobei als Gebissteil auch ein ganzes Ersatzgebiss gemeint sein kann. Alternativ oder zusätzlich kann die Gebissbild-Simulationsvorrichtung auch dazu benutzt werden, eine Veränderung der Zahnstellung zu simulieren und visualisieren, beispielsweise durch eine Zahnspangenbehandlung, mittels derer ein einzelner Zahn oder mehrere Zähne in eine andere Ausrichtung gezwungen werden. Dies kann beispielsweise auch dazu benutzt werden, zu simulieren, ob ein bislang schief stehender, herunterzuziehender Zahn in eine vorhandene Zahnlücke überhaupt hineinpasst oder ob ggf. weitere Maßnahmen vorgenommen werden müssen. Andererseits kann mit der Gebissbild-Simulationsvorrichtung auch ein Bleaching einzelner oder mehrerer Zähne simuliert werden. Alternativ oder zusätzlich können auch Maßnahmen und Veränderungen an dem Gebiss zugehörigen Geweben wie Kieferknochen oder Weichteile wie Lippen und Wangen simuliert werden.

Je nachdem, welche Zahnbehandlungsfunktion auszuführen ist, segmentiert die Segmentierungsvorrichtung die entsprechenden Zähne bzw. Gebiss- oder Zahnbereiche durch Identifikation der diese wiedergebenden Bildpixel, so dass der Segmentmanipulator die entsprechenden Bildpixel in Abhängigkeit der vorzunehmenden Behandlungsfunktion manipulieren kann. Der Segmentmanipulation kann hierbei bei die erhältliche Ist-Darstellung des Gebisses hinsichtlich Größe, Form, Farbe, Sättigung, Ausrichtung und/oder Position manipulieren.

Die vorzunehmende Zahnbehandlungsfunktion kann in vorteilhafter Weise manuell vorgegeben werden, beispielsweise durch Anklicken eines Funktionssymbols in einer Zahnbehandlungsfunktions-Menüleiste einer Steuerungsbildschirmdarstellung. Alternativ oder zusätzlich kann auch eine automatische Auswahl der vorzunehmenden Zahnbehandlungsfunktion in Abhängigkeit der Ist-Darstellung erfolgen. Enthält beispielsweise die Ist-Darstellung des Gebissbilds einen Zahn mit einer schräg abgeschlagenen Zahnkante, kann dies von einem Gebissbildanalysator automatisch erkannt und die entsprechende Zahnbehandlungsfunktion - im genannten Fall vorteilhafterweise das Einsetzen eines Zahnersatzes - vorgegeben werden.

Die Segmentierung eines zu behandelnden Zahnes bzw. Gebissteiles in der Ist-Darstellung desselben kann grundsätzlich auf verschiedene Art und Weise erfolgen. Erfindungsgemäß erfolgt die Segmentierung durch ein Bereichswachstumsverfahren, wobei ein Saatpunkt in dem zu segmentierenden Zahn bzw. Gebissabschnitt, oder auch außerhalb des zu segmentierenden Zahns bzw. Gebissabschnitts gesetzt wird. Von diesem Saatpunkt ausgehend werden benachbarte Bildpixel anhand zumindest eines Homogenitätskriteriums dahingehend beurteilt, ob sie noch zum selben Bildbereich oder nicht gehören.

Zusätzlich kann die Segmentierungsvorrichtung vorteilhafterweise auch mittels eines sog. Active Snake-Verfahrens arbeiten, bei dem eine aktive Konturbestimmungseinrichtung zur Bestimmung der Kontur eines zu segmentierenden Zahn- und/oder Gebissteils eine parametrisierte Kurve - die sogenannte snake - zunächst vorgibt und dann in Abhängigkeit gewisser Eigenschaften von Bildpunkten, die innerhalb und/oder außerhalb der Kurve liegen, die Form der Kurve verändert, sozusagen ausbeult und einbeult, zieht und streckt bzw. staucht und dergleichen. Insbesondere kann dabei als Kurve eine Umrisskurve vorgegeben werden, die näherungsweise bereits einer üblichen Zahnkontur entspricht, bspw. anhand der Durchschnittswerte abgespeicherte Zahnumrisslinien, wobei die vorgebbare Umrisskurve anhand verschiedener Kriterien wie beispielsweise die Lage des zu segmentierenden Zahns im Gebiss auswählbar ist, um bereits ausgangsmäßig eine größtmögliche Übereinstimmung zu haben. Die zunächst vorgegebene Schlange bzw. Kurve wird sodann vorteilhafterweise durch eine Gradientenvektor-Bestimmungseinrichtung zur Bestimmung von Gradientenvektoren anhand von Bildpunkteigenschaften eines von der parametrisierten Kurve umschlossenen Bildbereichs und/oder eines die parametrisierte Kurve umgebenden Bildbereichs verformt und somit in Abhängigkeit erfasster Eigenschaften des erzeugten Ist-Bildes der zu bearbeitenden Gebisses verformt. Hierzu kann eine in Abhängigkeit der bestimmten Gradientenvektoren arbeitende Konturformungseinrichtung zur Verformung der parametrisierten Kurve in Abhängigkeit der bestimmten Gradientenvektoren vorgesehen sein.

Zusätzlich kann die Segmentierung mittels eines zellulären Automaten erfolgen. Zelluläre oder auch zellulare Automaten dienen der Modellierung räumlich diskreter dynamischer Systeme, wobei die Entwicklung einzelner Zellen zum Zeitpunkt t + 1 primär von den Zellzuständen in einer vorgegebenen Nachbarschaft und vom eigenen Zustand zum Zeitpunkt t abhängt. Ein solcher Zellularautomat ist dabei insbesondere durch folgende Größen festgelegt:
- ein Raum R (Zellraum)
- eine endliche Nachbarschaft N
- eine Zustandsmenge Q
- eine lokale Überführungsfunktion δ: Q^{N} → Q

Der Zellraum besitzt hierbei eine gewisse Dimensionalität, er ist in der Regel 1-oder 2-dimensional, kann aber durchaus auch höherdimensional sein. Der Zellularautomat nimmt dabei durch seine globale Konfiguration eine Abbildung aus dem Zellraum in die Zustandsmenge vor, das heißt man ordnet jeder Zelle des Automaten einen Zustand zu. Der Übergang einer Zelle von einem Zustand (lokale Konfiguration) in den nächsten wird durch Zustandsübergangsregeln definiert, die deterministisch oder stochastisch sein können. Die Zustandsübergänge erfolgen für alle Zellen nach derselben Überführungsfunktion und gleichzeitig. Die Zellzustände können wie die Zeitschritte diskret sein. Nur wenige Zustandswerte reichen zur Simulation selbst hochkomplexer Systeme aus. Durch Bestimmung der genannten endlichen Nachbarschaft N kann der jeweils interessierende Bereich segmentiert werden. Auch bei der Segmentierung mittels eines zellulären Automaten kann von einem oder mehreren Saatpunkten ausgegangen werde, die innerhalb und/oder außerhalb des zu segmentierenden Bildabschnitts liegen können.

Als Homogenitätskriterium für die Segmentierung können hierbei verschiedene Bildpunkteigenschaften herangezogen werden. Insbesondere kann bei der Segmentierung von Zähnen vorteilhafterweise der Grauwert eines jeweiligen Bildpunktes analysiert werden, da sich die regelmäßig weißen Zähne von Nachbarkonturen gerade im Grauwert stark unterscheiden werden.

Alternativ oder zusätzlich kann als Homogenitätskriterium auch ein Farbton, eine Sättigung und/oder eine Helligkeit des jeweiligen Bildpunktes analysiert werden.

Vorteilhafterweise können mehrere Eigenschaften eines Bildpunktes als Homogenitätskriterium herangezogen werden. Nach einer vorteilhaften Weiterbildung der Erfindung kann das Bereichswachstumsverfahren durch den sog. RGB-Farbraum umfassend die Primärfarben Rot, Grün und Blau, genauer gesagt mittels eines Farbvektors, der die Primärfarben Rot, Grün und Blau eines Bildpunktes angibt, gesteuert werden. Alternativ oder zusätzlich kann auch der HSI-Farbraum bestehend aus dem Farbton, der Sättigung und der Helligkeit herangezogen werden. In diesem Fall wird ein den Farbton, die Sättigung und die Helligkeit angebender Farbvektor eines jeden Bildpunkts als Homogenitätskriterium verwendet.

Erfindungsgemäß ist eines Vergleichseinrichtung vorgesehen, mittels derer der Wert der untersuchten Eigenschaft eines jeweiligen Bildpunkts, insbesondere die Richtung und die Länge des zuvor genannten Farbvektors mit einem Referenzwert dieser Eigenschaft, insbesondere einem Referenzvektor verglichen wird und die sich ergebende Differenz des Vergleichs mit einem Schwellwert verglichen wird. Überschreitet die Abweichung ein zulässiges Maß, wird der Bildpunkt als nicht mehr zum selben Bildbereich zugehörig identifiziert, während umgekehrt bei nur kleinen, d.h. unter dem Schwellwert liegenden Abweichungen die umgekehrte Zuordnung getroffen wird.

Der für den genannten Vergleich herangezogene Vergleichswert, also insbesondere der Vergleichsvektor des jeweiligen Farbraums und/oder der entsprechende Grauwert kann grundsätzlich in verschiedener Art und Weise festgelegt werden. Nach einer besonders einfachen Ausbildung der Erfindung kann der Referenzwert des Homogenitätskriteriums der entsprechende Eigenschaftswert des Saatpunkts sein. Dem liegt die Überlegung zugrunde, dass der Saatpunkt regelmäßig in einem zentralen Bereich des jeweiligen Bildabschnitts gesetzt ist, der sicher dem jeweiligen Bereich zugehörig ist.

Um jedoch auch bei graduellen Veränderungen die Bereichsgrenze deutlich zu identifizieren, beispielsweise bei sich kontinuierlich verändernden Zahnverfärbungen die Zahngrenze sicher zu identifizieren, wird gemäß der Erfindung vorgesehen, dass der Referenzwert für das Homogenitätskriterium adaptiv festgelegt wird, beispielsweise dergestalt, dass der entsprechende Eigenschaftswert eines zuvor untersuchten und benachbarten Bildpixels herangezogen wird. Dabei bestimmt ein adaptiver Vergleichswertgenerator einen Mittelwert mehrerer Eigenschaftswerte mehrerer zuvor untersuchter Bildpixel. Dies können die unmittelbar benachbarten Bildpixel sein, wobei als zusätzliches Korrekturglied jedoch auch ein Mittelwert des entsprechenden Eigenschaftswerts aller zuvor untersuchten Bildpixel des Bereichs herangezogen wird. Hierdurch können einerseits graduelle Eigenschaftsänderungen wie Zahnverfärbungen berücksichtigt, andererseits jedoch Überlagerungen in Randbereichen nicht zu weit toleriert werden.

Der Bildanalysator weist einen Gewichtungsgeber zur Gewichtung einzelner Bildpunkte anhand ihrer Abweichung von einem zuvor bestimmten Mittelwert und/oder anhand ihres Abstands von dem Saatpunkt auf. Dem liegt die Überlegung zugrunde, dass einzelne Bildpunkte bei vorliegend bestimmter Eigenschaften sicherer als andere Bildpunkte zu dem gesuchten Bereich gehören und/oder für die Bestimmung des gesuchten. Bereichs wichtiger sind. Wird beispielsweise festgestellt, dass ein Bildpunkt hinsichtlich des ihn identifizierenden Farbraumvektors identisch mit dem zuvor bestimmten Mittelwert ist, kann mit relativ hoher Sicherheit davon ausgegangen werden, dass er zum selben Bereich gehört. Andererseits kann beispielsweise ein näher am Saatpunkt liegender Bildpunkt höher gewichtet werden als ein weiter entfernt liegender, da unter der Annahme, dass der Saatpunkt recht zentral in dem gesuchten Bereich liegt, näher daran liegende Punkte ebenfalls noch im gesuchten Bereich liegen. Gibt man diesen Punkten ein höheres Gewicht für die statistische Auswertung, insbesondere für die Mittelwertbildung bezüglich des Referenzwerts, kann eine genauere Bestimmung des zu segmentierenden Bereichs erfolgen.

Nach einer weiteren vorteilhaften Weiterbildung der Erfindung kann die Segmentierungsvorrichtung eine Bereichskanten-Bestimmungseinrichtung zur Bestimmung von Bereichskanten anhand eines Sprungs in der verglichenen Eigenschaft der Bildpunkte besitzen. Eine solche Bereichskanten-Bestimmungseinrichtung nutzt die Überlegung, dass zu segmentierende Zähne und/oder Gebissabschnitte durch an sich glatte Begrenzungskanten begrenzt werden, und zwar sowohl zum Zahnfleisch hin als auch zu einem jeweils benachbarten Zahn sowie darüber hinaus auch am freien Ende des Zahns, so dass anders als bei ausgefransten Konturen eine glatte Konturlinie als Begrenzung bestimmt werden muss. Bestimmt die Segmentierungsvorrichtung für einen Bildpunkt eine sprunghafte Veränderung des Homogenitätskriteriums im Vergleich zu benachbarten Bildpunkten, kann mit großer Wahrscheinlichkeit darauf geschlossen werden, dass ein solcher Bildpunkt ein Begrenzungspunkt der Kontur ist. Beispielsweise wird am Zahnfleischrand eine sprunghafte Änderung des Farbraums eintreten, da von der üblicherweise weißen Farbe des Zahns auf die rosa Farbe des Zahnfleischs übergegangen wird. Werden auf diese Weise zumindest einige Bildpunkte als Begrenzungspunkte identifiziert, kann die Zahnkontur sauber segmentiert werden, indem derartige Begrenzungspunkte miteinander durch eine gerade und/oder kontinuierliche Kurve verbunden werden. Mittels einer solchen Bestimmung von Bereichskanten können an einzelnen Stellen nicht sauber identifizierbare Konturränder und Ausreißer eliminiert werden und die Zahnkontur sauber festgelegt werden.

Alternativ oder zusätzlich kann in Weiterbildung der Erfindung eine Abgleichseinrichtung vorgesehen sein, mittels derer der jeweils segmentierte Zahn und/oder der jeweils segmentierte Gebissabschnitt mit einem gespeicherten Sollbild des zu segmentierenden Zahns und/oder Gebissbilds abgeglichen wird. Hierzu kann insbesondere ein statistisches Abweichungsmodell Verwendung finden, mittels dessen Abweichungen des segmentierten Zahns und/oder Gebissabschnitts von einem Mittelwert der gespeicherten Sollbilder bestimmt wird und/oder ein zulässiger Abweichungskorridor von diesem Mittelwert der gespeicherten Sollbilder vorgegeben wird. Insbesondere kann mittels eines solchen statistischen Modells die zu segmentierende Zahnkontur in die Sollkontur des jeweiligen Zahns bzw. Gebissabschnitts hineinwachsen, wodurch eine sauberere, weniger fehlerbehaftete Bereichsbestimmung erreicht wird.

Um insbesondere bei schwieriger zu erkennenden Bereichskanten dennoch eine sichere Bestimmung der Bereichsgrenzen zu erzielen, kann in Weiterbildung der Erfindung auch mit einer Annäherung von beiden Seiten her gearbeitet werden, d.h. es wird nicht nur der an sich auszuschneidende Bereich segmentiert, sondern auch der diesen Bereich umgebende, sozusagen negative Bereich. Insbesondere kann mit einem Bereichswachstumsverfahren von zwei Saatpunkten aus, von denen einer im zu segmentierenden Bereich liegt und einer außerhalb des zu segmentierenden Bereichs liegt, sozusagen aufeinander zu gearbeitet werden. Soll beispielsweise die obere Zahnreihe segmentiert werden, kann ein Saatpunkt in der Zahnreihe und ein Saatpunkt im Zahnfleisch gesetzt werden, so dass der Bereich der zu segmentierenden Zähne auf den Zahnfleischrand zuwächst und der diesen umgebende Zahnfleischbereich von oben her auf die Zahnfleisch-/Zahngrenze zuwächst.

Je nachdem, welche Zahnbehandlungsfunktion durchzuführen ist, kann die Bildmanipulationsvorrichtung verschiedene Werkzeuge aufweisen. In Weiterbildung der Erfindung kann die Bildmanipulationsvorrichtung insbesondere eine ZahnersatzBestimmungseinrichtung zur automatischen Bestimmung eines Ersatzzahnes in Abhängigkeit des segmentierten Bildausschnitts aufweisen. Soll beispielsweise ein einzelner Zahn ersetzt werden, bestimmt die Zahnersatzbestimmungseinrichtung automatisch anhand des segmentierten Bildausschnitts den am besten passenden Ersatzzahn, der hinsichtlich Kontur und/oder Farbe bestmöglich passt.

Um aus einer Vielzahl von gespeicherten Ersatzzahnmodellen den bestpassenden richtig auszuwählen, kann die Zahnersatzbestimmungseinrichtung eine Normierungseinrichtung aufweisen, mittels derer der segmentierte Zahn bzw. Gebissabschnitt zunächst auf eine Sollhöhe normiert wird, und zwar insbesondere auf einen Mittelwert der Höhenwerte aller Zähne eines gespeicherten Referenzmodells. Auf Basis dieser normierten Zahnhöhe wird dann mittels einer Konturvergleichsvorrichtung ein nächstkommender Referenzzahn bzw. ein nächstkommender Referenzgebissabschnitt aus einer Vielzahl von gespeicherten Referenzzähnen und/oder Referenzgebissabschnitten ausgewählt.

Vorteilhafterweise werden hierbei gespeicherte zweidimensionale Darstellungen von Referenzzähnen bzw. Referenzgebissabschnitten verwendet. Alternativ oder zusätzlich kann jedoch auch vorgesehen sein, dass die Zahnersatzbestimmungseinrichtung bzw. die Konturvergleichsvorrichtung Bilder bzw. Darstellungen aus 3D-Datenbanken verwendet und diese dreidimensionalen Bilder entsprechend transformiert, d.h. in eine zweidimensionale Bilddarstellung transformiert und mit der richtigen Ausrichtung des Zahns bzw. Gebissabschnittes in das System als 2D-Bild einfügt. Hierdurch kann eine Schnittstelle zu dreidimensional arbeitenden Systemen, 3D-Datenbanken und CAD-Systemen geschaffen werden. Im Sinne einer einfachen Ausbildung mit geringen Datenmengen und Rechenschritten ist vorteilhafterweise jedoch die Verwendung von zweidimensionalen Referenzdarstellungen vorgesehen.

Ist der jeweilige Ersatzzahn bzw. Gebissabschnitt ausgewählt, wird dessen Kontur automatisch an den segmentierten Bildausschnitt angepasst. Dies kann insbesondere durch Skalierung mit dem zuvor bestimmten Normierungsfaktor, mit dem der segmentierte Bildausschnitt auf den Mittelwert der Zahnsollhöhe normiert wurde, erfolgen. Alternativ oder zusätzlich kann auch die seitliche Kontur an die Kontur der Zahnlücke, in die der Ersatzzahn einzusetzen ist, angepasst werden. Bei der Ersatzzahnbehandlung und deren Visualisierung ist es insofern vorteilhaft, nicht nur den zu ersetzenden Zahn zu segmentieren, sondern auch die Zahnlücke zu segmentieren, insbesondere dadurch, dass die seitlichen Zähne, die die Zahnlücke begrenzen, segmentiert werden. Hierdurch können eventuell vorhandene Zahnlücken zwischen dem zu ersetzenden Zahn im Originalgebiss und den angrenzenden Zähnen berücksichtigt werden.

Die Konturanpassung kann vorteilhafterweise mittels eines vorab gespeicherten Statistikmodells erfolgen, das gespeicherte Konturmittelwerte als Sollkontur vorgibt. Alternativ dazu kann auch vorgesehen werden, dass der Ersatzzahn/ die Ersatzzähne aus dem Ist-Bild durch Verwendung des/der kontralateralen Zahnes/zähne durch Spiegelung abgeleitet wird.

Um die Visualisierung von Schwankungen bei der Bildgenerierung unabhängig zu machen und/oder unterschiedliche Belichtungsverhältnisse zu eliminieren, ist in Weiterbildung der Erfindung eine Kalibriervorrichtung vorgesehen, mittels derer die Ist-Darstellung des Gebisses hinsichtlich Größe und/oder Farbe und/oder Grauton kalibriert wird. Dies kann insbesondere dadurch erfolgen, dass auf dem zu erzeugenden Ist-Bild des zu behandelnden Gebisses Referenzobjekte mit abgebildet werden, deren Größe und/oder Farbton und/oder Helligkeit und/oder Sättigung und/oder Grauton bekannt ist. Insbesondere kann ein Sortiment von Referenzzähnen und/oder eine Längenskala wie beispielsweise ein Lineal mit abgebildet werden, so dass einerseits der zu segmentierende Zahn bzw. Gebissabschnitt hinsichtlich Farbton durch Zuordnung zum nächstkommenden Referenzmodell sowie andererseits hinsichtlich seiner Abmessungen wie Zahnhöhe und Zahnbreite bestimmbar ist.

Die Erfindung wird nachfolgend anhand eines bevorzugten Ausführungsbeispiels und zugehöriger Zeichnungen näher erläutert. In den Zeichnungen zeigen:
- Fig. 1:: eine schematische Seitenansicht der Ist-Bilderzeugungsvorrichtung mit der Kopfpositioniervorrichtung zur Positionierung des Kopfes eines Patienten sowie der relativ hierzu montierten Kamera,
- Fig. 2:: eine schematische Frontansicht des von der Kopfpositioniervorrichtung positionierten Patientenkopfes sowie der bei der Erzeugung des Ist-Bilds in das Bild platzierten Kalibriervorrichtung,
- Fig. 3:: die Gebissbild-Simulationsvorrichtung mit einer Ist-Darstellung des Gebisses in seinem Ist-Zustand, und
- Fig. 4:: eine schematische Seitenansicht der Ist-Bilderzeugungsvorrichtung mit einem Patienten in Seitenansicht sowie der relativ hierzu montierten Kamera zur Erzeugung eines Ist-Bilds des Patientenkopfs, um zusammen mit der Frontansicht eine 3-D Simulation des Gebissbilds zu erzeugen..

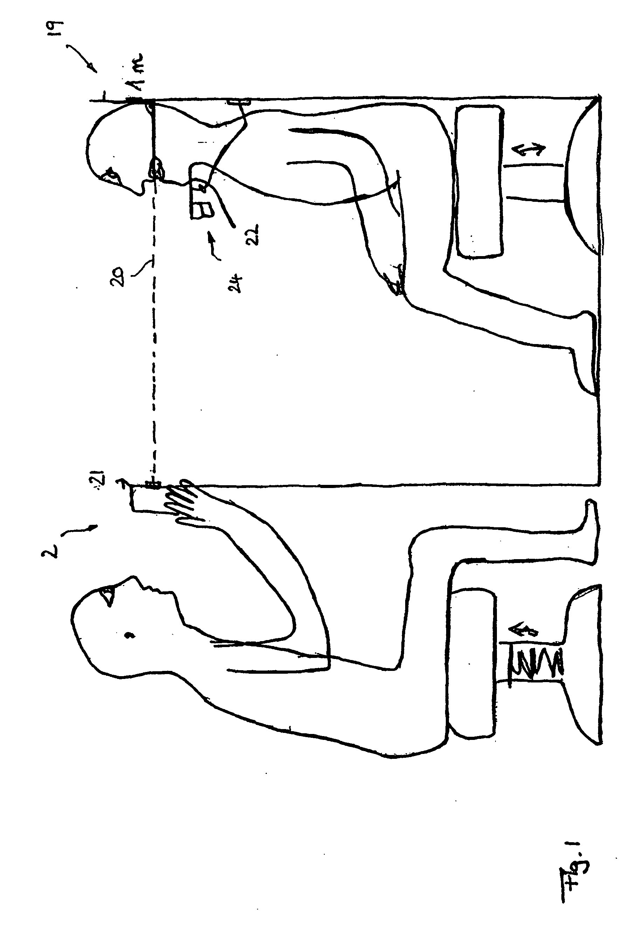

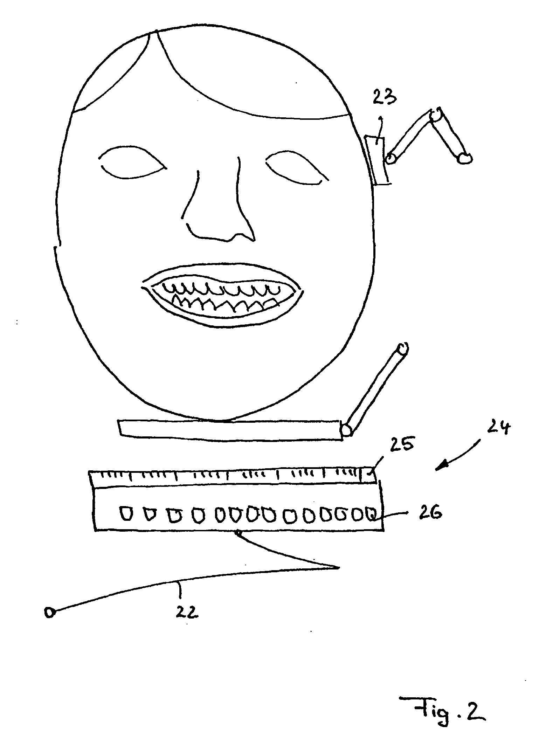

Die in den Zeichnungen dargestellte Gebissbild-Simulationsvorrichtung 1 umfasst die in Fig. 1 gezeigte Kopfpositioniervorrichtung 19, zu der relativ in vorbestimmter Position eine Kamera 21 montiert ist. Die Kopfpositioniervorrichtung 19 umfasst in der gezeichneten Ausführung vorteilhafterweise eine höhen- und winkeleinstellbare Kinnstütze 22, zumindest eine einstellbare Seitenstütze 23 sowie eine ebenfalls einstellbare Hinterkopfstütze 24, vgl. Figuren 1 und 2. Mittels dieser einstellbaren Stützen kann die Kopfpositioniervorrichtung 19 den Kopf des Patienten derart ausrichten, dass die Okklusionsebene 20, die von den Zahnkauflächen bei geschlossenem Gebiss definiert wird, exakt ausgerichtet werden kann, und zwar derart, dass das Objektiv der Kamera 21 in der genannten Okklusionsebene 20 zu liegen kommt und die Bildhorizontale in der genannten Okklusionsebene 20 liegt. Durch ein derartiges Ausrichten der Kopfposition kann erzielt werden, dass das Gebiss in einer exakt identischen Position abgebildet wird, und zwar insbesondere in der Position, in der auch die Referenzzähne der gespeicherten Referenzzahnmodelle abgebildet sind.

Wie Fig. 2 zeigt, wird bei der Generierung der Ist-Darstellung eine Kalibriervorrichtung 24 umfassend einen Längenmaßstab 25 in Form eines Lineals sowie einen Satz von Referenzzähnen 26 in das Bild eingeblendet. Die genannte Kalibriervorrichtung 24 kann hier beispielsweise an einem abwinkelbaren Haltearm 27 befestigt sein, mittels derer der Längenmaßstab 25 sowie die Referenzzähne 26 unter der Kinnstütze 22 positioniert werden können.

Um den Kopf exakt in der gewünschten Position ausrichten zu können, kann die Kopfpositioniervorrichtung 19 vorteilhafterweise weitere Hilfsmittel aufweisen, wie beispielsweise ein Beißspatel, auf das der Patient zu beißen hat und mittels dessen die Position des Kopfes exakt in der genannten Weise ausgerichtet werden kann, während die diversen einstellbaren Stützen eingestellt werden. Die variable Einstellbarkeit hat den Hintergrund, dass das Gebiss nicht immer in exakt derselben Ausrichtung innerhalb der Kopfkontur positioniert ist.

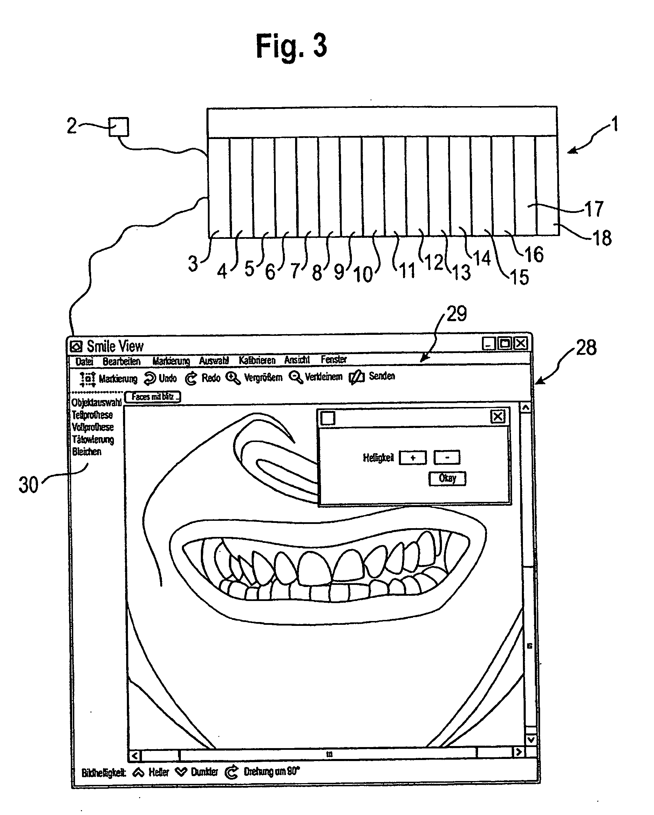

Mittels der Kamera 21 wird sodann ein digitales Bild des Gebisses erzeugt, das auf einem Bildschirm 28 der Gebissbildsimulationsvorrichtung 21 digital angezeigt werden kann, vgl. Fig. 3. Die Ist-Darstellung ist hierbei in einen Menürahmen 29 eingeblendet, über den diverse Steuerbefehle eingegeben werden können. Wie Fig. 3 zeigt, kann beispielsweise durch Anklicken der linken Menüleiste 30 die gewünschte Zahnbehandlungsfunktion ausgewählt werden, wie beispielsweise eine Zahnersatzfunktion durch Anklicken des Befehls "Teilprothese" bzw. "Vollprothese".

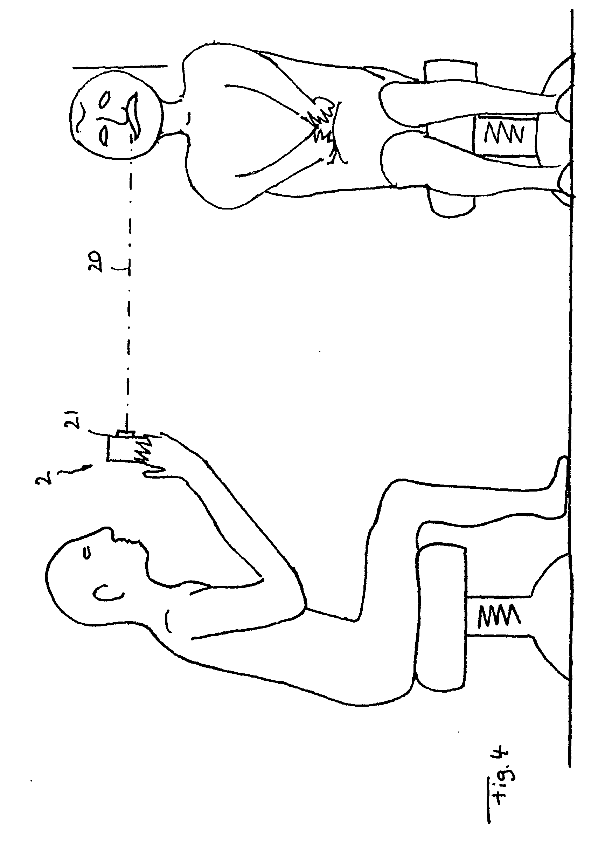

Um eine einfache und schnelle Datenverarbeitung zu erreichen, kann ein zweidimensionales Gebissbild erzeugt bzw. simuliert werden. Für diesen Fall ist die Erzeugung eines Ist-Bilds des Patienten in Frontansicht gemäß Figur 1 ausreichend. Soll jedoch eine dreidimensionale Gebissbildsimulation erzeugt werden, wird vorteilhafterweise zumindest eine zweite Ist-Darstellung des Gebisses bzw. des Patientenkopfs erzeugt, und zwar vorteilhafterweise eine Seitenansicht, wie dies Figur 4 zeigt. Auch hier wird der Kopf mittels der Kopfpositioniereinrichtung 19 in die entsprechend ausgerichtete Position gebracht, so dass die Linse der Kamera in der Okklusionsebene liegt. Aus den beiden 2-D-Bildern kann dann die gewünschte 3-D-Darstellung errechnet werden.

Soll beispielsweise die gesamte obere Zahnreihe ersetzt werden, ist in der Ist-Darstellung der die obere Zahnreihe wiedergebende Bildabschnitt auszuschneiden. Hierzu umfasst die Gebissbild-Simulationsvorrichtung 1 eine Segmentierungsvorrichtung 5, die die obere Zahnreihe segmentiert. Die Segmentierungsvorrichtung 5 kann hierbei nach einer Ausführung der Erfindung eine Bereichswachstums-Bestimmungseinrichtung 8 aufweisen, die von einem Saatpunkt ausgehend in der eingangs beschriebenen Weise einen entsprechenden Bildbereich anhand eines Homogenitätskriteriums oder mehrerer solcher Homogenitätskriterien, wie zuvor beschrieben, identifiziert. Der Saatpunkt kann hierbei grundsätzlich manuell gesetzt werden, beispielsweise durch Anklicken eines der Zähne durch Positionieren eines Cursors und Anklicken des entsprechenden Punkts. Alternativ kann der Saatpunkt auch automatisch gesetzt werden, wozu die Segmentierungsvorrichtung 5 einen Saatpunktgenerator 7 besitzen kann, der beispielsweise einen Zahn anhand der Bildhelligkeit und auch der vorbekannten Information identifizieren kann, dass die Kauebene bzw. Okklusionsebene in der Ebene des Kameraobjektivs und damit in einem bestimmten Bildabschnitt liegt.

Die Bereichswachstums-Bestimmungseinrichtung 8 analysiert hierbei von dem Saatpunkt ausgehend Pixel für Pixel das Bild mit einem Bildpunktanalysator 9, der Bildpunkteigenschaften wie beispielsweise den Farbraumvektor eines Bildpunkts analysiert und mittels einer Vergleichseinrichtung 10 mit einem entsprechenden Referenzwert vergleicht. Der Referenzwert kann durch einen Vergleichswertgenerator 11 adaptiv bestimmt werden. Insbesondere kann wie eingangs erläutert durch einen Mittelwertbildner 12 der Vergleichswert anhand der Mittelwerte zuvor bestimmter Bildpunkteigenschaften angepasst werden.

Ist von der Segmentiervorrichtung 5 die Darstellung der oberen Zahnreihe segmentiert, wird diese zunächst mittels einer Normierungseinrichtung 16 auf eine normierte Zahnhöhe normiert, woraufhin eine Konturvergleichsvorrichtung 17 aus einer Datenbank, in der diverse Zahn- und Gebissmodelle abgespeichert sind, das am nächstkommende Gebissmodell für die zu ersetzende obere Zahnreihe ausgewählt wird. Wird nur die obere Zahnreihe ersetzt, wird dabei das entsprechende Referenzzahnmodell ausgewählt, das hinsichtlich Farbe der unteren Zahnreihe am nächsten kommt. Alternativ oder zusätzlich kann das ausgewählte Referenzzahnmodell hinsichtlich seines Farbtons entsprechend angepasst werden.

Das ausgewählte Referenzzahnmodell wird ebenso hinsichtlich seiner Form und Größe an die Kontur des segmentierten Gebissabschnitts angepasst. Eine Kontur-anpassungsvorrichtung 18 streckt bzw. staucht die Darstellung des ausgewählten Referenzzahnmodells derart, dass es sich bestmöglich in den segmentierten Bildabschnitt einfügt.

## Patentansprüche

1. Gebissbild-Simulationsvorrichtung zur Simulation eines Gebissbilds zur Behandlungsergebnis-Visualisierung und/oder zur Generierung von Daten für die Herstellung von Zahnersatzteilen, mit einer Ist-Bilderzeugungsvorrichtung (2) zur Erzeugung einer Ist-Darstellung des Gebisses in seinem Ist-Zustand, einer Bild-Manipulationsvorrichtung (3) zur Manipulation von Bilddaten der Ist-Darstellung anhand einer vorgebbaren Gebiss- und/oder Zahnbehandlungsfunktion, und einer Soll-Bilderzeugungsvorrichtung (4) zur Erzeugung einer Soll-Darstellung des Gebisses in seinem Soll-Zustand anhand der Ist-Darstellung und der manipulierten Bilddaten, wobei die Bild-Manipulationsvorrichtung (3) eine Segmentierungsvorrichtung (5) zur automatischen Segmentierung eines Zahns und/oder eines Gebissteils in der Ist-Darstellung in Abhängigkeit der vorgegebenen Gebiß- und/oder Zahnbehandlungsfunktion sowie einen Segmentmanipulator (6) zur automatischen Manipulation des segmentierten Bildausschnitts in Abhängigkeit der vorgegebenen Gebiß- und/oder Zahnbehandlungsfunktion aufweist, wobei die Segmentierungseinrichtung (5) einen Saatpunktgenerator (7) zur Erzeugung eines Saatpunkts innerhalb und/oder außerhalb des zu segmentierenden Zahns und/oder Gebissabschnitts sowie eine Bereichswachstums-Bestimmungseinrichtung (8) zur Bestimmung eines vom Saatpunkt aus wachsenden Bildbereichs anhand zumindest eines Homogenitätskriteriums für einzelne Bildpunkte des Bildbereichs aufweist, wobei die Bereichswachstums-Bestimmungseinrichtung (8) einen Bildpunktanalysator (9) zur Bestimmung zumindest einer Eigenschaft eines dem Saatpunkt oder einem zuvor analysierten Bildpunkt benachbarten Bildpunkts, eine Vergleichseinrichtung (10) zum Vergleichen der bestimmten Eigenschaft eines jeweiligen Bildpunkts mit einem Wert dieser Eigenschaft für den Saatpunkt und/oder den zuvor analysierten Bildpunkt sowie zum Zuweisen des Bildpunkts zum Bildbereich in Abhängigkeit des Vergleichs, insbesondere in Abhängigkeit des Abgleichs der Abweichung mit einem Schwellwert, aufweist, **dadurch gekennzeichnet, dass** die Vergleichseinrichtung (10) einen adaptiven Vergleichswertgenerator (11) zur adaptiven Bestimmung eines Vergleichswerts für die zu vergleichende Eigenschaft anhand der zuvor bestimmten Eigenschaftswerte zuvor untersuchter Bildpunkte aufweist, wobei der adaptive Vergleichswertgenerator (11) einen Mittelwertbildner (12) zur Bildung eines Mittelwerts der zuvor bestimmten Eigenschaftswerte zuvor untersuchter Bildpunkte sowie einen Gewichtungsgeber zur Gewichtung einzelner Bildpunkte anhand ihrer Abweichung von einem zuvor bestimmten Mittelwert aufweist.

2. Gebissbild-Simulationsvorrichtung nach dem vorhergehenden Anspruch, wobei die zumindest eine Eigenschaft einen Grauwert, einen Farbton, eine Sättigung und/oder eine Helligkeit des Bildpunkts umfasst, und/oder als Homogenitätskriterium ein die Primärfarben Rot, Grün und Blau angebender Farbvektor und/oder ein den Farbton, die Sättigung und die Helligkeit angebender Farbvektor eines jeden Bildpunkts vorgesehen ist.

3. Gebissbild-Simulationsvorrichtung nach einem der vorhergehenden Ansprüche, wobei die Segmentierungseinrichtung (5) eine Bereichskanten-Bestimmungseinrichtung (13) zur Bestimmung von Bereichskanten anhand eines Sprungs in der verglichenen Eigenschaft der Bildpunkte und zur Verbindung solchermaßen identifizierter Bildpunkte aufweist.

4. Gebissbild-Simulationsvorrichtung nach einem der vorhergehenden Ansprüche, wobei die Segmentierungseinrichtung (5) eine aktive Konturbestimmungseinrichtung zur Bestimmung der Kontur eines zu segmentierenden Zahn- und/oder Gebißteils mittels einer parametrisierten Kurve aufweist, wobei die aktive Konturbestimmungseinrichtung eine Gradientenvektor-Bestimmungseinrichtung zur Bestimmung von Gradientenvektoren anhand von Bildpunkteigenschaften eines von der parametrisierten Kurve umschlossenen Bildbereichs und/oder eines die parametrisierte Kurve umgebenden Bildbereichs, und eine Konturformungseinrichtung zur Verformung der parametrisierten Kurve in Abhängigkeit der bestimmten Gradientenvektoren aufweist.

5. Gebissbild-Simulationsvorrichtung nach einem der vorhergehenden Ansprüche, wobei die Segmentierungseinrichtung (5) eine Abgleichseinrichtung (14) zum Abgleich des segmentierten Zahns und/oder Gebissabschnitts mit einem gespeicherten Soll-Bild des zu segmentierenden Zahns und/oder Gebissbilds aufweist, wobei die Abgleichseinrichtung (14) ein statistisches Abweichungsmodel zur Bewertung von Abweichungen des segmentierten Zahns und/oder Gebissabschnitts von einem Mittelwert der gespeicherten Soll-Bilder und/oder zur Vorgabe eines zulässigen Abweichungskorridors aufweist.

6. Gebissbild-Simulationsvorrichtung nach einem der vorhergehenden Ansprüche, wobei die Bereichswachstums-Bestimmungseinrichtung (8) von zwei Saatpunkten ausgehend, von denen einer im zu segmentierenden Bereich und einer im nicht zu segmentierenden Bereich generiert ist, zwei aufeinander zuwachsende Bildbereiche und deren Trennlinie voneinander bestimmt.

7. Gebissbild-Simulationsvorrichtung nach einem der vorhergehenden Ansprüche, wobei die Bild-Manipulationsvorrichtung (3) eine Zahnersatz-Bestimmungseinrichtung (15) zur Bestimmung eines Ersatzzahnes in Abhängigkeit des segmentierten Bildausschnitts aufweist, wobei die Zahnersatz-Bestimmungseinrichtung (15) eine Normierungseinrichtung (16) zur Normierung des segmentierten Zahns und/oder Gebissabschnitts auf eine Soll-Höhe, insbesondere auf einen Mittelwert der Höhenwerte aller Zähne eines gespeicherten Referenzmodells, aufweist, und eine Konturvergleichs-Vorrichtung (17) zur Bestimmung eines nächstkommenden Referenzzahnes und/oder Referenz-Gebissabschnitts aus einer Vielzahl von gespeicherten Referenzzähnen und/oder Referenz-Gebissabschnitten aufweist.

8. Gebissbild-Simulationsvorrichtung nach dem vorhergehenden Anspruch, wobei die gespeicherten und/oder zu verwendenden Referenzzähne und/oder gespeicherten und/oder zu verwendenden Referenz-Gebissabschnitte Zahn-und/oder Gebissdarstellungen umfassen, die den jeweiligen Zahn- und/oder Gebissabschnitt in derselben Ausrichtung und in demselben Abstand zeigen, in der und in dem der Zahn- und/oder Gebissabschnitt in der Ist-Darstellung der Ist-Bilderzeugungsvorrichtung (2) gezeigt ist, wobei die Zahnersatz-Bestimmungseinrichtung (15) eine Kontur-Anpassungsvorrichtung (18) zur automatischen Anpassung der Kontur des ausgewählten Referenzzahnes und/oder Referenz-Gebissabschnitts an den segmentierten Bildausschnitt aufweist, wobei die Kontur-Anpassungsvorrichtung (18) ein vorab gespeichertes Statistikmodel zur Anpassung der Zahnkontur anhand gespeicherter Konturmittelwerte umfasst.

9. Gebissbild-Simulationsvorrichtung nach einem der vorhergehenden Ansprüche, wobei die Bild-Manipulationsvorrichtung (3) eine Kalibriervorrichtung zur Kalibrierung der Ist-Darstellung des Gebisses hinsichtlich Größe und/oder Farbe und/oder Grauton aufweist.

10. Gebissbild-Simulationsvorrichtung nach einem der vorhergehenden Ansprüche, wobei die Bild-Manipulationsvorrichtung (3) den Segmentmanipulator beinhaltet, der nach erfolgter Auswahl der Behandlungsfunktion und Segmentierung die Manipulation der Ist-Darstellung des Gebisses hinsichtlich Größe, Form, Farbe, Sättigung, Ausrichtung und/oder Position ermöglicht.

## Claims

1. A dentition image simulation apparatus for simulating a dentition image for visualising a treatment result and/or for generating data for manufacturing denture parts, having an actual image generation apparatus (2) for generating an actual representation of the dentition in its actual state, having an image manipulation apparatus (3) for manipulating image data of the actual representation with reference to a predefinable dentition and/or tooth treatment function, and having a desired image generation apparatus (4) for generating a desired representation of the dentition in its desired state with reference to the actual representation and the manipulated image data, wherein the image manipulation apparatus (3) has a segmentation apparatus (5) for the automatic segmentation of a tooth and/or of a dentition part in the actual representation in dependence on the predefined dentition and/or tooth treatment function and has a segment manipulator (6) for the automatic manipulation of the segmented image section in dependence on the predefined dentition and/or tooth treatment function, wherein the segmentation device (5) has a seed point generator (7) for generating a seed point inside and/or outside the tooth and/or dentition section to be segmented and has a region growth determination apparatus (8) for determining a image region growing out of the seed point with reference to at least one homogeneity criterion for individual picture elements of the image region, wherein the region growth determination device (8) has a picture element analyser (9) for determining at least one property of a picture element adjacent to the seed point or to a previously analysed picture element, has a comparator (10) for comparing the determined property of a respective picture element with a value of this property for the seed point and/or with the previously analysed picture element as well as for assigning the picture element to the image region in dependence on the comparison, in particular in dependence on the comparison of the deviation with a threshold value,
**characterised in that**
the comparator (10) has an adaptive comparison value generator (11) for the adaptive determination of a comparison value for the property to be compared with reference to the previously determined property values of previously examined picture elements, wherein the adaptive comparison value generator (11) has an averaging device (12) for forming an average of the previously determined property values of previously examined picture elements and has a weighting encoder for weighting individual picture elements with reference to their deviation from a previously determined average value.

2. A dentition image simulation apparatus in accordance with the preceding claim, wherein the at least one property includes a grey value, a colour shade, a saturation and/or a brightness of the picture element; and/or wherein a colour vector indicating the primary colours red, green and blue is provided as a homogeneity criterion; and/or wherein a colour vector of each picture element indicating the colour hue, the saturation and the brightness is provided.

3. A dentition image simulation apparatus in accordance with one of the preceding claims, wherein the segmentation device (5) has a region edge determination device (13) for determining region edges with reference to a jump in the compared properties of the picture elements and for connecting such identified picture elements.

4. A dentition image simulation apparatus in accordance with one of the preceding claims, wherein the segmentation device (5) has an active contour determination device for determining the contour of a tooth part and/or dentition part to be segmented by means of a parameterised curve, wherein the active contour determination device has a gradient vector determination device for determining gradient vectors with reference to picture element properties of an image region surrounded by the parameterised curve and/or of an image region surrounding the parameterised curve, and has a contour forming device for forming the parameterised curve in dependence on the determined gradient vectors.

5. A dentition image simulation apparatus in accordance with one of the preceding claims, wherein the segmentation device (5) has a comparison device (14) for comparing the segmented tooth and/or dentition section with a stored desired image of the tooth and/or dentition image to be segmented, wherein the comparison device (14) has a statistical deviation model for evaluating deviations of the segmented tooth and/or dentition section from an average of the stored desired images and/or for predefining a permitted deviation corridor.

6. A dentition image simulation apparatus in accordance with one of the preceding claims, wherein the region growth determination device (8), starting from two seed points of which one is generated in the region to be segmented and one is generated in the region not to be segmented, determines two image regions growing towards one another and their separation line from one another.

7. A dentition image simulation apparatus in accordance with one of the preceding claims, wherein the image manipulation apparatus (3) has a denture determination device (15) for determining an artificial tooth in dependence on the segmented image section, wherein the denture determination device (15) has a standardising device (16) for standardising the segmented tooth and/or dentition section to a desired height, in particular to an average of the height values of all teeth of a stored reference model, and has a contour comparator (17) for determining a closest reference tooth and/or reference dentition section from a plurality of stored reference teeth and/or reference dentition sections.

8. A dentition image simulation apparatus in accordance with the preceding claim, wherein the stored reference teeth and/or the reference teeth to be used and/or the stored reference dentition sections and/or reference dentition sections to be used include tooth representations and/or dentition representations which show the respective tooth section and/or dentition section in the same alignment and at the same interval in which the tooth section and/or dentition section is shown in the actual representation of the actual image generation apparatus (2), wherein the denture determination device (15) has a contour adaptation apparatus (18) for the automatic adaptation of the contour of the selected reference tooth and/or reference dentition section to the segmented image section, wherein the contour adaptation apparatus (18) includes a previously stored statistical model for adapting the tooth contour with reference to stored contour averages.

9. A dentition image simulation apparatus in accordance with one of the preceding claims, wherein the image manipulation apparatus (3) has a calibration apparatus for calibrating the actual representation of the dentition with respect to size and/or colour and/or grey shade.

10. A dentition image simulation apparatus in accordance with one of the preceding claims, wherein the image manipulation apparatus (3) includes the segment manipulator which allows the manipulation of the actual representation of the dentition with respect to size, shape, colour, saturation, alignment and/or position after a selection of the treatment function and segmentation has taken place.

## Revendications

1. Dispositif de simulation d'une image de dentition destiné à la simulation d'une image de dentition en vue de la visualisation d'un résultat de soins et/ou de la génération de données pour la fabrication d'éléments de rechange dentaires, avec un dispositif de génération d'image réelle (2) en vue de la création d'une représentation réelle de la dentition dans son état réel, un dispositif de manipulation d'image (3) en vue de la manipulation de données d'imagerie de la représentation réelle à l'aide d'une fonction de soins dentaires et/ou prothétiques prescriptible, et un dispositif de création d'image théorique (4) en vue de la création d'une représentation théorique de la dentition dans son état théorique à l'aide de la représentation réelle et des données d'imagerie manipulées, le dispositif de manipulation d'image (3) présentant un dispositif de segmentation (5) en vue de la segmentation automatique d'une dent et/ou d'une partie de la dentition dans la représentation réelle en fonction de la fonction de soins dentaires et/ou prothétiques prescrite ainsi qu'un manipulateur de segment (6) en vue de la manipulation automatique de la section d'imagerie segmentée en fonction de la fonction de soins dentaires et/ou prothétiques prescrite, le dispositif de segmentation (5) présentant un générateur de point de semence (7) en vue de la génération d'un point de semence à l'intérieur et/ou à l'extérieur de la dent et/ou section de dentition à segmenter ainsi qu'un dispositif de détermination de la croissance d'une zone (8) en vue de la détermination d'une zone d'imagerie croissant à partir du point de semence à l'aide d'au moins un critère d'homogénéité pour certains points d'image de la zone d'imagerie, le dispositif de détermination de la croissance d'une zone (8) présentant un analyseur de point d'image (9) en vue de la détermination d'au moins une propriété d'un point d'image avoisinant le point de semence ou un point d'image analysé auparavant, un dispositif de comparaison (10) en vue de la comparaison de la propriété déterminée d'un point d'image respectif avec une valeur de cette propriété pour le point de semence et/ou le point d'image analysé auparavant ainsi qu'en vue d'affecter le point d'image à la zone d'imagerie en fonction de la comparaison, notamment en fonction du réglage de la divergence avec une valeur seuil, **caractérisé en ce que** le dispositif de comparaison (10) présente un générateur de valeur de comparaison adaptatif (11) en vue de la détermination adaptative d'une valeur de comparaison pour la propriété à comparer à l'aide des valeurs de propriétés déterminées auparavant de points d'image examinés auparavant, le générateur de valeur de comparaison adaptatif (11) présentant un formateur de valeur moyenne (12) en vue de la formation d'une valeur moyenne des valeurs de propriétés déterminées auparavant de points d'image examinés auparavant ainsi qu'un transmetteur de pondération en vue de la pondération de points d'image individuels à l'aide de leur divergence par rapport à une valeur moyenne déterminée auparavant.

2. Dispositif de simulation d'une image de dentition selon la revendication précédente, au moins l'une des propriétés comprenant une valeur de gris, une teinte, une saturation et/ou une clarté du point d'image, et/ou comme critère d'homogénéité, un vecteur chromatique indiquant les couleurs primaires rouge, vert et bleu et/ou un vecteur chromatique indiquant la teinte, la saturation et la clarté de chacun des points d'image étant prévu.

3. Dispositif de simulation d'une image de dentition selon une quelconque des revendications précédentes, le dispositif de segmentation (5) présentant un dispositif de détermination des bordures de zones (13) en vue de la détermination de bordures de zones à l'aide d'un saut dans la propriété comparée des points d'image et en vue de la jonction de points d'image identifiés de la sorte.

4. Dispositif de simulation d'une image de dentition selon une quelconque des revendications précédentes, le dispositif de segmentation (5) présentant un dispositif actif de détermination des contours en vue de la détermination du contour d'une partie de dent et/ou de dentition à segmenter au moyen d'une courbe paramétrée, le dispositif actif de détermination des contours présentant un dispositif de détermination des vecteurs de gradients en vue de la détermination des vecteurs de gradients à l'aide des propriétés de points d'image d'une zone d'imagerie entourée par la courbe paramétrée et/ou d'une zone d'imagerie entourant la courbe paramétrée, et un dispositif de formage des contours pour le formage de la courbe paramétrée en fonction des vecteurs de gradients déterminés.

5. Dispositif de simulation d'une image de dentition selon une quelconque des revendications précédentes, le dispositif de segmentation (5) présentant un dispositif de réglage (14) en vue du réglage de la dent et/ou section de dentition segmentée avec une image théorique enregistrée de la dent à segmenter et/ou de l'image de dent, le dispositif de réglage (14) présentant un modèle de déviation statistique en vue de l'évaluation des déviations de la dent et/ou section de dentition segmentée d'une valeur moyenne des images théoriques enregistrées et/ou en vue de la prescription d'un corridor de déviation admissible.

6. Dispositif de simulation d'une image de dentition selon une quelconque des revendications précédentes, le dispositif de détermination de la croissance d'une zone (8) étant déterminé en partant de deux points de semence, dont l'un est généré dans la zone à segmenter et l'un dans la zone non à segmenter, deux zones d'imagerie s'accumulant l'une sur l'autre et leur ligne de séparation l'une de l'autre.

7. Dispositif de simulation d'une image de dentition selon une quelconque des revendications précédentes, le dispositif de manipulation d'image (3) présentant un dispositif de détermination de prothèse dentaire (15) en vue de la détermination d'une dent de rechange en fonction de la section d'imagerie segmentée, le dispositif de détermination de prothèse dentaire (15) présentant un dispositif de normalisation (16) en vue de la normalisation de la dent et/ou section de dentition segmentée à une hauteur théorique, notamment à une valeur moyenne des valeurs de hauteur de toutes les dents d'un modèle de référence enregistré, et un dispositif de comparaison des contours (17) présentant, en vue de la détermination d'une dent de référence et/ou section de dentition de référence la plus proche parmi une multitude de dents de référence et/ou sections de dentition de référence enregistrées.

8. Dispositif de simulation d'une image de dentition selon la revendication précédente, les dents de référence enregistrées et/ou à utiliser et/ou les sections de dentition de référence enregistrées et/ou à utiliser comprenant des représentations de dent et/ou de dentition, qui montrent la section de dent et/ou de dentition respective dans le même alignement et dans le même écart que dans ceux où la section de dent et/ou de dentition est montrée dans la représentation réelle du dispositif de génération d'image réelle (2), le dispositif de détermination de prothèse dentaire (15) présentant un dispositif d'adaptation des contours (18) en vue de l'adaptation automatique du contour de la dent de référence et/ou la section de dentition de référence sélectionnée sur la section d'imagerie segmentée, le dispositif d'adaptation des contours (18) comprenant un modèle statistique enregistré au préalable en vue de l'adaptation du contour de dent à l'aide des valeurs moyennes de contours enregistrées.

9. Dispositif de simulation d'une image de dentition selon une quelconque des revendications précédentes, le dispositif de manipulation d'image (3) présentant un dispositif de calibrage en vue du calibrage de la représentation réelle de la dentition en matière de taille et/ou de couleur et/ou de teinte grise.

10. Dispositif de simulation d'une image de dentition selon une quelconque des revendications précédentes, le dispositif de manipulation d'image (3) contenant le manipulateur de segment, qui, après la sélection effectuée de la fonction de soins et la segmentation, permet la manipulation de la représentation réelle de la dentition en matière de taille, de forme, de couleur, de saturation, d'alignement et/ou de position.
